# EUROPEAN PATENT APPLICATION

(11) **EP 2 246 341 A1**
(43) Date of publication of application: **03.11.2010**
(21) Application number: 09000928.3
(22) Date of filing: 23.01.2009
(51) Int. Cl.: C07D 307/82, C07C 49/172, C07C 251/52

(54) **Process for preparing 2-Alkyl-3-aroyl-5-nitro-benzofurans**

(71) Applicant: Lonza Ltd., 4052 Basel (CH)
(72) Inventor: The designation of the inventor has not yet been filed

(57) **Abstract**

The present invention relates to a process for the preparation of a compound of formula 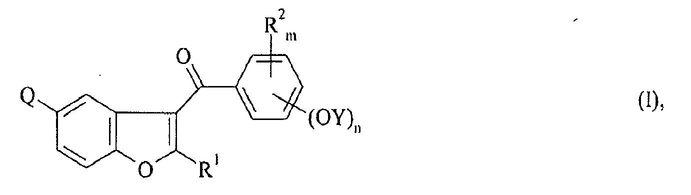 wherein R¹ is selected from C₁₋₆-alkyl, C₃₋₆-cycloalkyl and aralkyl, and
wherein R² at each occurrence independently is halogen or C₁₋₆-alkyl, and m is an integer from 0 to 4, Q is selected from halogen, -NO₂ and -NR³R⁴, wherein R³ and R⁴ are independently selected from hydrogen, C₁₋₆-alkyl, C₁₋₆-cycloalkyl, aryl, aralkyl, mesyl and tosyl, or wherein R³ and R⁴ together form a C₄₋₆-alkylene group, Y at each occurrence is hydrogen or a hydroxy protection group W that can be hydrolytically cleaved under acidic conditions, and n is an integer from 1 to 3, and n is an integer from 1 to 3, with the proviso that n and m together are not greater than 5.

## Description

The present invention relates to a process for the preparation of a compound of formula 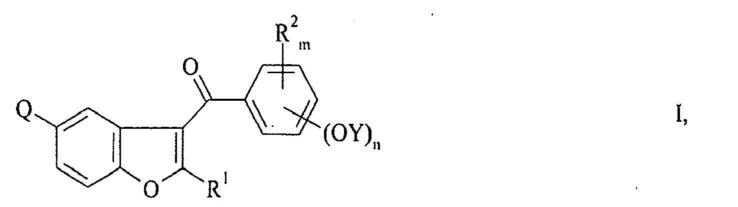 wherein R¹ is selected from C₁₋₆-alkyl, C₃₋₆-cycloalkyl and aralkyl,
R² at each occurrence independently is halogen or C₁₋₆-alkyl, and m is an integer from 0 to 4,
Q is selected from halogen, -NO₂ and -NR³R⁴, wherein R³ and R⁴ are independently selected from hydrogen, C₁₋₆-alkyl, C₃₋₆-cycloalkyl, aryl, aralkyl, mesyl and tosyl, or wherein R³ and R⁴ together form a C₄₋₆-alkylene group,
Y at each occurrence is hydrogen or a hydroxy protection group W that can be hydrolytically cleaved under acidic conditions, and n is an integer from 1 to 3.
and n is an integer from 1 to 3, with the proviso that n and m together are not greater than 5.

Here and hereinbelow the term halogen represents an atom selected from fluorine, chlorine, bromine and iodine.

Here and hereinbelow the term "Cₛ₋ₜ-alkyl" represents a linear or branched alkyl group, having s to t carbon atoms, wherein s and t are integers. C₁₋₆-alkyl represents for example methyl, ethyl, propyl, isopropyl, butyl, isobutyl, *sec*-butyl, *tert*-butyl, pentyl and hexyl.

Here and hereinbelow the term "Cₛ₋ₜ-alkoxy" represents a linear or branched alkoxy group having s to t carbon atoms, wherein s and t are integers. C₁₋₆-alkoxy represents for example methoxy, ethoxy, propoxy, isopropoxy, butoxy, isobutoxy, *sec*-butoxy, *tert*-butoxy, pentyloxy and hexyloxy.

The term "C₃₋ₜ-cycloalkyl" represents a cycloaliphatic group having 3 to t carbon atoms.
C₃₋₁₀-cycloalkyl represents for example mono- and polycyclic ring systems such as cyclopropyl, cyclobutyl, cyclopentyl, cyclohexyl, cycloheptyl, cyclooctyl, adamantyl or norbornyl.

The term aryl represents an aromatic group, optionally substituted with one or more halogen atoms, amino groups, and/or optionally substituted C₁₋₆-alkyl, C₁₋₆-alkoxy or di-C₁₋₆-alkylainino groups. For example C₆₋₂₀-aryl represents phenyl, naphthyl and derivatives thereof as outlined above.

The term aralkyl represents an alkyl group substituted with an aromatic group, wherein the alkyl group is linear C₁₋₈-alkyl and the aryl group is selected from phenyl, naphthyl, furanyl, thienyl, benzo[b]furanyl, benzo[b]thienyl, each of them optionally being substituted with one or more halogen atoms, amino groups, and/or optionally substituted C₁₋₆-alkyl, C₁₋₆-alkoxy or di-C₁₋₆-alkylamino groups.

Here and hereinbelow the term di-C₁₋₆-alkylamino represents an amino group substituted with two containing two C₁-C₆ alkyl groups, the latter optionally being substituted with one or more halogen atoms.

Particularly dronedarone, known from US-A-5,223,510, 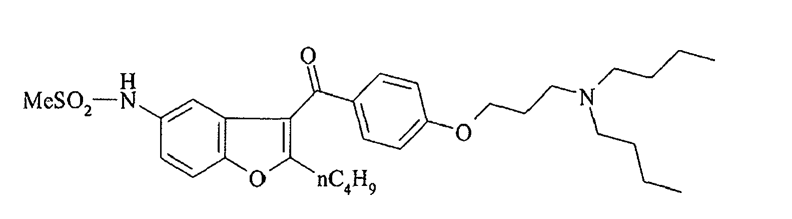 a compound according to formula I, wherein R¹ = *n*-butyl, Y = W = -(CₚH₂ₚ)-Z, wherein p = 3, Z = NR¹²R¹³, wherein R¹² = R¹³ = *n*-C₄H₉, m = 0, n = 1 and Q = NR³R⁴, wherein R³ = mesyl and R⁴ = hydrogen, is known to be pharmaceutically active as potassium channel blocker used for the treatment of heart arrhythmia, angina pectoris and thrombosis.

In known processes for example of EP-A-471609 and WO-A-02/048078, a phenolic compound of formula I wherein R¹ is butyl, OY is 4-hydroxy and Q is nitro, a valuable precursor of dronedarone, can be obtained by acylation of 2-butyl-5-nitrobenzofuran with 4-anisoyl chloride. The main drawback of this method is the need of a Friedel-Crafts catalyst (AlCl₃ or SnCl₄) for the acylation step. Friedel-Crafts acylations using these catalysts result in the production of large amounts of metal hydroxide waste. Furthermore, the need for a deprotection step (demethylation step) to obtain a free phenol function requires large amounts of a strong Lewis acid (AlCl₃ or similar), which also contributes to the waste production. Finally, the initial 2-butyl-5-nitrobenzofuran is not a commodity but has to be produced by a multi-step process.

In an alternative process according to FR-A-2864536 or EP-A-1116719, 2-butyl-5-nitrobenzofuran-3-carbonyl chloride is arylated with anisole. The alternative route also involves a Friedel-Crafts reaction followed by a demethylation step, both steps using AlCl₃ or similar Lewis acids as reagents. As a further drawback, this method results in the formation of regioisomeric by-products, due to the limited selectivity of the Friedel-Crafts acylation. Moreover, the preparation of 2-butyl-5-nitrobenzofuran-3-carbonyl chloride is a delicate process requiring 3 chemical steps. The difference between both processes is the time of introduction of the carbonyl group to which R² is attached.

The technical problem to be solved was to provide an alternative method for the preparation of 2-alkyl-3-aroyl-5-nitro-benzofurans in high regioselectivity. A further object was to provide a robust and secure process for the preparation of suitable amounts for the pharmaceutical industry. Another object was to establish a new route avoiding the use of Friedel-Crafts reactions requiring Lewis acids such as AlCl₃ which often a negative environmental potential. Furthermore, the general concept should start with easily available compounds and should contain few reaction steps, allowing the synthesis of a wide variety of products.

The problem has been solved by the process of claim 1.

Claimed is a process for the preparation of a compound of formula 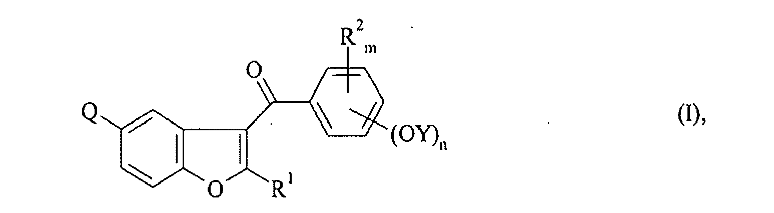 wherein R¹ is selected from C₁₋₆-alkyl, C₃₋₆-cycloalkyl and aralkyl,
R² at each occurrence independently is halogen or C₁₋₆-alkyl, and m is an integer from 0 to 4,
Q is selected from halogen, -NO₂ and -NR³R⁴, wherein R³ and R⁴ are independently selected from hydrogen, C₁₋₆-alkyl, C₃₋₆-cycloalkyl, aryl, aralkyl, mesyl and tosyl, or wherein R³ and R⁴ together form a C₄₋₆-alkylene group,
Y at each occurrence is hydrogen or a hydroxy protection group W that can be hydrolytically cleaved under acidic conditions, and n is an integer from 1 to 3, with the proviso that n and m together are not greater than 5,
comprising the steps of
(i) reacting a compound of formula 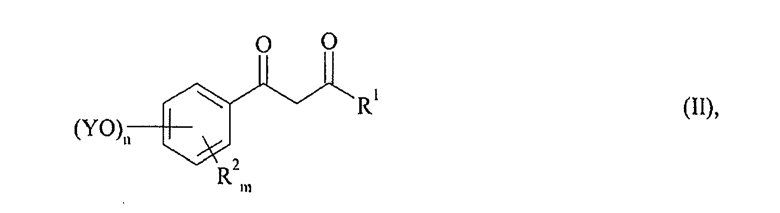 wherein R¹, R², Y, n and m are as defined above,
   with a compound of formula 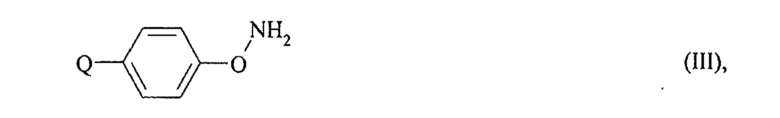 wherein Q is as defined above, or a salt thereof,
   optionally in the presence of an acid, to obtain the compound of formula 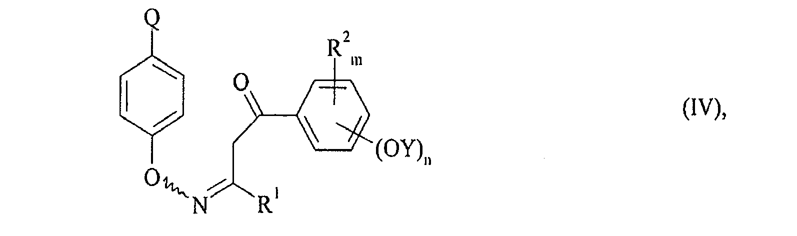 wherein R¹, R², Y, Q, n and m are as defined above, and
(ii) subjecting the compound of formula IV to an oxime rearrangement (ring closure), optionally in the presence of an acid, to obtain the compound of formula I.

In a preferred embodiment n is 1 and OY is a *para*-oriented substituent regarding the carbonyl group attached to the aromatic ring. In a further preferred embodiment m is 0 and R² thus not exists.

The present process has several advantageous features which make it appealing for industrial application. It comprises a step (i), reacting an appropriate 1,3-diketone of formula II with an O-arylhydroxylamine of formula III to form an O-aryloxime of formula IV bearing only one oxime group, while an carbonyl group is attached to an aryl residue followed by a subsequent step (ii) of an oxime rearrangement. In particular step (ii) produces almost no regioisomer, involves no Friedel-Crafts reaction and therefore requires no metal catalyst. Finally, in case of phenolic residues it requires no seperate deprotection step for the phenol function. Provided is also a process for the preparation of the 1,3-diketones used in step (i). The starting compounds are easy available commodities.

In the case of using an O-aryloxime of the formula IV, the compound of formula I can be obtained with complete or almost complete regioselectivity of at least 95%, preferably of at least 97%, even more preferably of at least 99%.

Regardless of the residue -OY in the compound II, the O-aryloxime IV, wherein Q, Y, R¹, R², m and n are as defined above, is formed as an intermediate. This compound, having two different residues attached to the oxime carbon atom, wherein one comprises an carbonylaryl group provides a high selectivity regarding the subsequent oxime rearrangement affording a compound of formula I up to 99.9% or even higher.

Suitable hydroxy protection groups W can be cleaved in the presence of an acid and are inert towards basic conditions. The latter is important if compound II shall be prepared according to the present invention. Examples are amino, silyl and optionally further substituted alkoxy protection groups.

Thus, among others, a particularly suitable hydroxy protection group W is selected from
(a) -C(R⁵)(CH₂R⁶)-O-CH₂R⁷, wherein
   R⁵ is hydrogen or C₁₋₆-alkyl,
   R⁶ is selected from hydrogen, C₁₋₆-alkyl and C₃₋₆-cycloalkyl, and R⁷ is selected from hydrogen, C₁₋₆-alkyl, C₃₋₆-cycloalkyl and aryl, each alkyl, cycloalkyl or aryl of R⁵, R⁶ and R⁷ optionally and independently being substituted with one or more halogen atoms;
   or R⁶ and R⁷ together are -CH₂- or -(CH₂)₂- and thus are part of an 5- or 6-membered heterocyclic ring, or
(b) -SiR⁸R⁹R¹⁰, wherein R⁸, R⁹ and R¹⁰ are independently selected from C₁₋₆-alkyl,
   C₃₋₆-cycloalkyl, aryl and aralkyl, or
(c) -(CₚH₂ₚ)-Z, wherein p is an integer from I to 6 and Z is H or -SR¹¹, wherein R¹¹ is selected from C₁₋₆-alkyl, C₃₋₆-cycloalkyl, aryl and aralkyl, or Z is -NR¹²R¹³, wherein R¹² and R¹³ are independently selected from C₁₋₆-alkyl, C₃₋₆-cycloalkyl, aryl and aralkyl, or R¹² and R¹³ together form a C₄₋₆-alkylene group.

In a preferred embodiment according to (a), W is selected from tetrahydrofuranyl, tetrahydropyranyl, 1-ethoxyethyl (EEO), 1-methyl-1-methoxyethyl or 1-methyl-1-benzyloxyethyl. In a preferred embodiment W is 1-ethoxyethyl.

In a further preferred embodiment according to (b), the hydroxy protection group W is a -SiR⁸R⁹R¹⁰ group, wherein each residue R⁸, R⁹ and R¹⁰ are as defined above, which can be hydrolized easily, even in the presence of a small acid amount. Most preferred silyl groups are trimethylsilyl and *tert*-butyldimethylsilyl groups.

In another preferred embodiment according to (c), the hydroxy protection group is -(CₚH₂ₚ)-Z, wherein Z and p are as defined above. When -(CₚH₂ₚ)-Z groups are used, preferably the compound II already comprises an appropriate side-chain of the final product already in place. For example, in the case of Dronedarone, using a 4-[3-(*N,N*-dibutylamino)propyll-oxy] protection group, no protection-deprotection reactions are necessary. This alternative route produces a further advanced intermediate, namely a direct precursor of the active pharmaceutical ingredient (api).

Another suitable -(CₚH₂ₚ)-Z group, wherein Z is hydrogen and p is 1 to 6, i.e. wherein OY is alkoxy, such as methoxy, ethoxy, propyloxy or butyloxy, the intermediate or final deprotection of the alkoxy group can be accomplished by means of AlCl₃, BCl₃, fuming HCl, pyridinium hydrochloride, and other strong acids. Using branched, preferably tertiary, alkoxy groups, protection and deprotection can be carried out at moderate acidic conditions as outlined below. (3)
It is possible but not necessary to isolate compound of formula IV. Thus, it is possible to carry out both the formation of the oxime compound VI and the subsequent oxime rearrangement as a one-pot process.

The oxime rearrangement to obtain the compound of formula I can be thermally and/or catalytically induced, i.e. simply by heating and or in the presence of an acid, respectively. In the presence of an acid the reaction can be accomplished at a lower temperature and more rapidly.

Thus, in a preferred embodiment, the oxime rearrangement of the compound of formula IV is carried out in the presence of an acid catalyst.

Preferably the acid catalyst is selected from strong anhydrous acids which promote both the condensation of step (i) and the subsequent rearrangement of the oxime compound IV of step (ii) to obtain the benzofurans of formula I.

Preferably, the acid catalyst is selected from anhydrous mineral acids such as HBr, HCl, HBF₄, Lewis acids such as BF₃ etherate, TiCl₄, and organic acids such as methanesulfonic acid, trifluoroacetic acid and aliphatic acids. Among aliphatic acids, formic acid is particularly preferred both as solvent and acid catalyst. Formic acid allows carrying out the reaction at the lowest temperature and the product crystallizes directly from the reaction mixture.

Optionally, the oxime rearrangement, regardless whether step (ii) is performed as an isolated process or not, can be carried out in a solvent such as ethyl acetate, butyl acetate, ethanol, nitroethane, and organic acids such as methanesulfonic acid, trifluoroacetic acid and aliphatic acids. Preferably the solvent mainly consists of the acid catalyst, more preferably mainly consists of formic acid or acetic acid.

Depending on the presence and the kind of the catalytic activity of the acid the oxime rearrangement can be carried out by room temperature or even at temperatures below 0 °C. Preferably the temperature is set between -20 to +150 °C.

Off compounds of formula I, wherein Y is different from hydrogen, the respective hydroxy protection group can be hydrolyzed easily by applying acidic conditions and further work-up to obtain the respective phenol compound. Preferably, the hydrolytically cleavage is carried out using acetic or formic acid.

The oxime rearrangement of compound IV provides an excellent regioselectivity of the compounds of formula I. Thus, we also claim the use of compounds of formula IV, wherein R¹ is selected from C₁₋₆-alkyl, C₃₋₆-cycloalkyl and aralkyl,
R² at each occurrence independently is halogen or C₁₋₆-alkyl, and m is an integer from 0 to 4,
Q is selected from halogen, -NO₂ and -NR³R⁴, wherein R³ and R⁴ are independently selected from hydrogen, C₁₋₆-alkyl, C₃₋₆-cycloalkyl, aryl, aralkyl, mesyl and tosyl, or wherein R³ and R⁴ together form a C₄₋₆-alkylene group,
Y at each occurrence is hydrogen or a hydroxy protection group W that can be hydrolytically cleaved under acidic conditions, and n is an integer from 1 to 3, with the proviso that n and m together are not greater than 5,
for the preparation of a compound of formula I.

Particularly preferred for said use W is selected from
(a) -C(R⁵)(CH₂R⁶)-O-CH₂R⁷, wherein
   R⁵ is hydrogen or C₁₋₆-alkyl,
   R⁶ is selected from hydrogen, C₁₋₆-alkyl and C₃₋₆-cycloalkyl, and R⁷ is selected from hydrogen, C₁₋₆-alkyl, C₃₋₆-cyclloalkyl and aryl, each alkyl, cycloalkyl or aryl of R⁵, R⁶ and R⁷ optionally and independently being substituted with one or more halogen atoms;
   or R⁶ and R⁷ together are -CH₂- or -(CH₂)₂- and thus are part of an 5- or 6-membered heterocyclic ring, or
(b) -SiR⁸R⁹R¹⁰, wherein R⁸, R⁹ and R¹⁰ are independently selected from C₁₋₆-alkyl, C₃₋₆-cycloalkyl, aryl and aralkyl, or
(c) -(CₚH₂ₚ)-Z, wherein p is an integer from 1 to 6 and Z is H or -SR¹¹, wherein R¹¹ is selected from C₁₋₆-alkyl, C₃₋₆-cycloalkyl, aryl and aralkyl, or Z is -NR¹²R¹³, wherein R¹² and R¹³ are independently selected from C₁₋₆-alkyl, C₃₋₆-cycloalkyl, aryl and aralkyl, or R¹² and R¹³ together form a C₄₋₆-alkylene group.

Further claimed is the use of a compound of formula I obtained by the process as disclosed above, wherein R¹, R² Q, n, and m are as defined in claim 1, for the preparation of a medicament.

In a preferred embodiment said use is **characterized in that** the medicament is a medicament for therapeutic application in heart arrhythmia, angina pectoris and/or thrombosis.

As mentioned above, a further aspect of the present invention covers the preparation of the 1,3-dicarbonyl compound of formula II out of commercially easily available compounds. The compounds of formula II, wherein W at each occurrence is as defined in options (a), (b) or (c) above are not known in the literature and therefore both the process to prepare compound II as well as the compound itself are of interest.

Claimed is a process for the preparation of a compound of formula 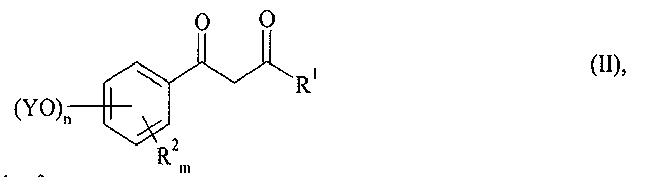 wherein R¹, R², Y, n and m are as defined in claim 1, comprising reacting a compound of formula 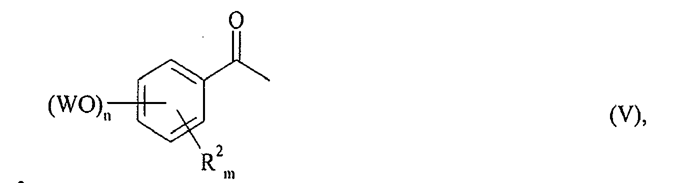 wherein R², W, n and m are as defined in claim 1, in the presence of a base, with a compound of formula 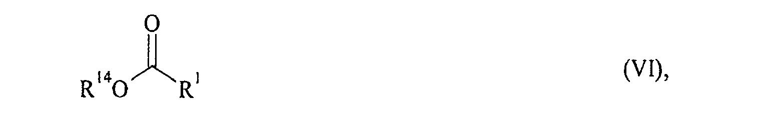 wherein R¹ is as defined in claim 1 and R¹⁴ is selected from the group consisting of C₁₋₆-alkyl, C₃₋₆-cycloalkyl and aralkyl, and
optionally hydrolytically cleaving W in the presence of an acid, to obtain a compound of formula II, wherein, Y is hydrogen.

In a preferred embodiment of the process above, W is
(a) -C(R⁵)(CH₂R⁶)-O-CH₂R⁷, wherein
   R⁵ is hydrogen or C₁₋₆-alkyl,
   R⁶ is selected from hydrogen, C₁₋₆-alkyl and C₃₋₆-cycloalkyl, and R⁷ is selected from hydrogen, C₁₋₆-alkyl, C₃₋₆-cycloalkyl and aryl, each alkyl, cycloalkyl or aryl of R⁵, R⁶ and R⁷ optionally and independently being substituted with one or more halogen atoms;
   or R⁶ and R⁷ together are -CH₂- or -(CH₂)₂- and thus are part of an 5- or 6-membered heterocyclic ring, or
(b) -SiR⁸R⁹R¹⁰, wherein R⁸, R⁹ and R¹⁰ are independently selected from C₁₋₆-alkyl, C₃₋₆-cycloalkyl, aryl and aralkyl, or
(c) -(CₚH₂ₚ)-Z, wherein p is an integer from 1 to 6 and Z is H or -SR¹¹, wherein R¹¹ is selected from C₁₋₆-alkyl, C₃₋₆-cycloalkyl, aryl and aralkyl, or Z is -NR¹²R¹³, wherein R¹² and R¹³ are independently selected from C₁₋₆-alkyl, C₃₋₆-cycloalkyl, aryl and aralkyl, or R¹² and R¹³ together form a C₄₋₆-alkylene group.

Particularly preferred the compound of formula 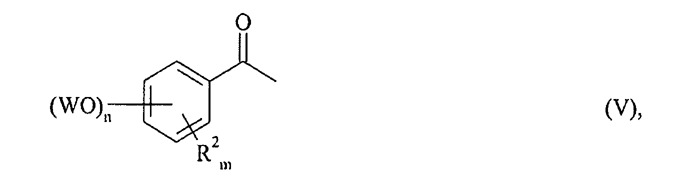 is prepared in a Claisen condensation by reacting a compound of formula, 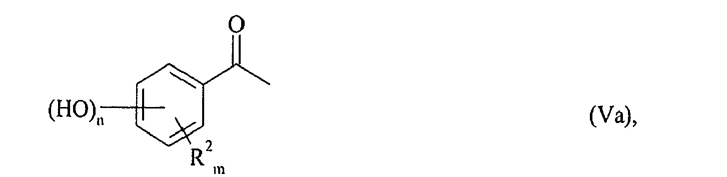 wherein R², n and m are as defined above, with at least n molar equivalents of
(i) a compound of formula 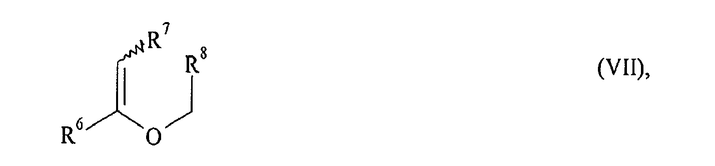 wherein R⁶, R⁷ and R⁸ are as defined above, to obtain a compound of formula 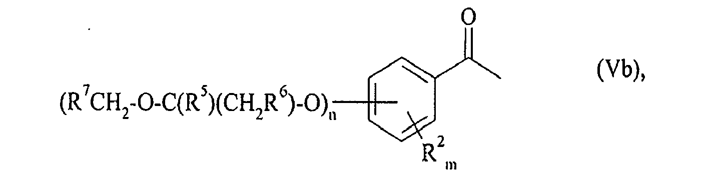 wherein R², R⁶, R⁷ and R⁸, m and n are as defined above, or
(b) a silylating agent comprising at least one group of the formula

   R⁸R⁹R¹⁰Si- (VIII),

   wherein R⁸, R⁹ and R¹⁰ are as defined in claim 2, to obtain a compound of formula 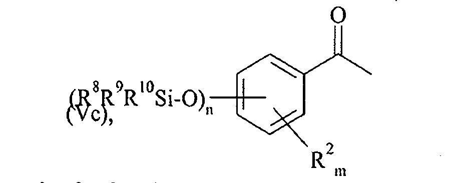 wherein R², R⁸, R⁹, R¹⁰, m and n are as defined above, or
(c) a compound of formula

   T-(CₚH₂ₚ)-Z (IX),
wherein T is selected from mesyl, tosyl, chlorine, bromine and iodine, and p and Z are as defined above, to obtain a compound of formula 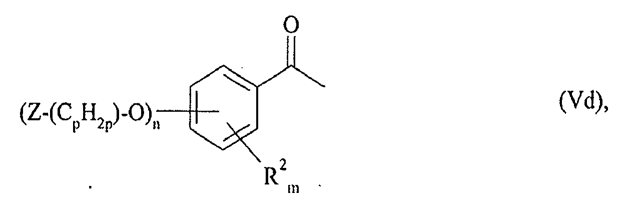 wherein R², Z, m, n and p are as defined above.

A particular feature of the inventive process is the ease of optionally removal of the protecting group, which can be carried out at any time from compounds II, IV or I. Particularly preferred it is carried out during the work-up of the Claisen condensation reacting compounds of formula V and VI, thus requiring no additional operation. Especially the ethoxyethyl group is cleaved off during work-up of the reaction mixture obtained in the Claisen condensation upon acidification, which is necessary when the 1,3-diketone of formula II shall be isolated as a neutral compound. Depending on the ease of removal of the hydroxy protection group work-up conditions can be selected in order to maintain or to cleave the protection group.

Another particular feature of the inventive process is that the process of preparing compounds II, IV and finally I, starting from compounds Vb, Vc or Vd, can be carried out as a one-pot process. Even more it is possible to start a one-pot process directly from compound Va.

Optionally the reaction sequence can be directly started from protected ketone of formula V, wherein (-OW) or (-O(CₚH₂ₚ)-Z) is selected from methoxy, ethoxy and linear or branched C₃₋₆-alkoxy, optionally being substituted with one or more halogen atoms. Accordingly, in that case (-OW) or (-O(CH₂)ₚ-Z) of the compounds of formula II also will be selected from methoxy, ethoxy and linear or branched C₃₋₆-alkoxy, each optionally being substituted with one or more halogen atoms.

In a preferred embodiment, protecting the ketone Va is carried out at a temperature from 0 to 30 °C, more preferred from 0 to 10 °C, and particularly preferred at about 5 °C.

A suitable solvent for protecting the ketone Va as outlined above is of medium polarity, preferably is selected from ethyl acetate, dioxane, tetrahydrofuran (THF), isobutyronitrile (IBN) and mixtures thereof. THF is the most preferred solvent to give fast conversion rates. It can be used also in a mixture with other solvents.

Preferably, the protection of the hydroxy group of the ketone Va is carried out in the presence of an acid catalyst. Strong acids such as H₂SO₄ and methanesulfonic acid are the most preferred acid catalysts, preferably said catalyst is selected from HCl, H₂SO₄, H₃PO₄, methanesulfonic acid, BF₃ etherate, trifluoroacetic acid, formic acid, acetic acid and mixtures thereof.

Preferably, the reaction mixture is quenched by addition of a moderate base. More preferably the base is a nitrogen base selected from the group consisting of trialkylamine, pyridine and imidazole. Particularly preferred the base is a alkylamine, more preferably trimethylamine or triethylamine. It appears that an incomplete reaction can be driven to completion while removing solvent under reduced pressure, preferably in the presence of trimethylammonium mesylate or tosylate.

While reacting a vinyl compound of formula VII with compound Va a ketal protecting group is formed. In a preferred mode the compound of formula VII comprising a carbon-carbon double bond and which is able to undergo such ketal reaction.

Particularly preferred compound VII is selected from 2,3-dihydrofuran (DHF), 2,3-dihydropyran (DHP), ethyl vinyl ether (EVE), methyl 2-propenyl ether (MPE) or benzyl 2-propenyl ether (BPE). Even more preferred, compound VII is EVE.

It could be shown that separate dosing of the compound of formula VII and the catalyst, both dissolved in the solvent, preferably in THF, is preferred to optimize the reaction in view of conversion, reduction of catalyst and compound of formula VII. In a preferred embodiment a moderate molar excess of the compound of formula VII compared to the number (n) of hydroxy groups attached to the compound of formula VII is used. Complete conversion in case of the reaction of p-hydroxyacetophenone with ethyl vinyl ether (EVE) could be obtained with about 40% molar excess of EVE.

Commonly used silylating agents for phenol protection are for example (Me₃Si)₂NH, (Me₃Si)₂NAc, Me₃SiCl, N,O-bis(trimethylsilyl)acetamide, (Me₃Si)₂O and *tert*-butyldimethylsilyl chloride. The O-silylation of 4-hydroxyacetophenone using (Me₃Si)₂NH is described in Firouzabadi, H. et al., J. Chem. Soc. Perkin Transactions 1, 23; 2002; 2601-2604.

In cases wherein compound Va is reacted with a compound IX, wherein Z is hydrogen and p is 1 or 2, i.e. wherein OW and thus OY is methoxy or ethoxy, intermediate or final deprotection of methoxy or ethoxy groups can be accomplished by means of AlCl₃, BCl₃, fuming HCl, pyridinium hydrochloride, and other strong acids.

When Z represents a dialkylamino or alkylthio group the group -(CₚH₂ₚ)- normally is a linear group. With Z is H, and p = 1 to 6, -(CₚH₂ₚ)- represents several branched hydroxy protection groups of the formula -(C₃H₆)- to -(C₆H₁₂)-. In a preferred embodiment Z-(CₚH₂ₚ)-O- is a *tert-*alkoxy group, which can be cleaved under mild acidic conditions, particularly preferred is *tert*-butyl or *tert*-amyl.

In terms of effort necessary for protection/deprotection both methoxy and ethoxy as Z-(CₚH₂ₚ)-O-groups have certain a disadvantage compared to *tert*-butyl, *tert*-amyl but also to any other hydroxy protection group according to the alternatives (a) to (c), as mentioned above. In terms of reactivity and selectivity of compounds II and III to compound IV and subsequent oxime rearrangement to compound I, both methoxy and ethoxy have no detrimental influence. Thus, methoxy and ethoxy hydroxy protecting groups can be used in the present processes.

Regardless of the kind of hydroxy protection group W, the reaction of compound V, i.e. Vb, Vc, Vd or any other suitable derivative of Va, with compound VI can be carried out without the addition of a solvent.

The compound of formula VI, at least if used in excess, which is needed for the Claisen condensation, is able to dissolve the reaction mixture. Thus, no addition of a further solvent is necessary. An advantageous side effect is that carrying the Claisen reaction without a solvent broadens the possibility to choose an appropriate solvent for work-up without the need of solvent exchange. If a solvent is used, the most appropriate solvent comprises isobutylnitril, either neat or as a mixture.

The Claisen condensation has a low reaction enthalpy and also doesn't need to be carried out under heating. It could be shown that heating is only recommended after complete addition of the reaction partners. The addition can be carried out a t a temperature from -10 to +30 °C. When a solvent is used, the addition is conducted at about 0 °C. In that case, preferably after the addition is completed, the reaction mixture is heated to about 80 to 100 °C to complete the reaction. When no solvent is used the reaction can be carried out at a room temperature, preferably at about 20 °C, and no additional heating is required. Typically the yield of compound II is about 90 to 95 mol-% compared to compound V.

It could be shown that the Claisen condensation of protected or unprotected hydroxy acetophenones with methyl valerate proceeds fairly quickly using potassium *tert*-butylate in isobutyronitrile. A complete conversion takes place within 30 to 60 minutes at 80 °C, and the crude product is obtained in excellent yield by a simple process. This procedure avoids using crown ethers or other phase transfer catalysts. It allows using the crude condensation product directly for further conversion.

Preferably, the base used in the Claisen condensation is a strong base, more preferably it is potassium *tert*-butylate. The R¹⁴-OH by-products of the reaction further increase the solubility of the base in the reaction mixture.

In a particularly preferred embodiment the Claisen condensation is performed reacting methyl valerate with 4-ethoxyethyl acetophenone in the presence of potassium *tert*-butylate in isobutyronitrile or even without a solvent.

In a preferred embodiment the resulting dicarbonyl compounds of formula II, wherein Y is not hydrogen, W can be hydrolytically cleaved in the presence of a diluted inorganic or organic proton acid prior to reacting with compound III. Preferably said proton acid is selected from the group consisting of sulfuric acid, hydrochloric acid, formic acid and acetic acid to obtain the compound of formula II, wherein Y is hydrogen.

The required O-arylhydroxylamines of formula III can be prepared by either one of several known procedures. In a preferred embodiment the hydroxylamine of formula III is obtained by reacting a *N-tert*-butoxycarbonyl (*N*-BOC) derivative of a corresponding amino compound in the presence of an acid, preferably an inorganic or organic proton acid. In a further preferred embodiment the hydroxylamine of formula III is obtained by reacting phthalimide derivative a corresponding amino compound under hydrazinolysis under anhydrous conditions.

Hydroxylamines are known to undergo a condensation reaction with simple 1-aryl-alkane-1,3-diones regioselectively at the 3 position only. Surprisingly, this is also the case using the diketone of formula II. Said condensation reaction occurs under mild conditions and is promoted by acid catalysts which favour the elimination of water. A variety of acid catalysts can be used to effect the condensation.

In a preferred embodiment, compound IX comprises an appropriate side chain of the drug product already in place. For example in the case of the production of Dronedarone, in a preferred embodiment compound V is 4-[3-(*N,N*-dibutylamino)propyl-1-oxy]acetophenone. In that case, no protection-deprotection reactions are necessary. This alternative route produces a further advanced intermediate, namely the direct precursor of the active pharmaceutical ingredient (api) obtained after oxime rearrangement of compound IV.

According to the present invention several new compounds can be prepared. Among these, claimed is a compound of formula 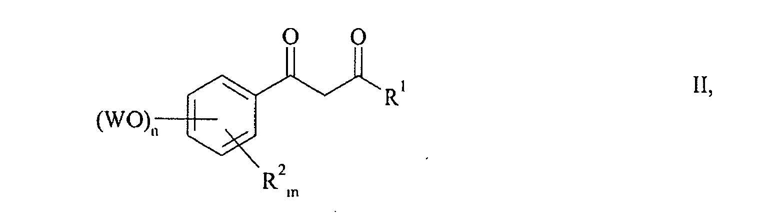 wherein R¹ is selected from C₁₋₆-alkyl, C₃₋₆-cycloalkyl and aralkyl,
R² at each occurrence independently is halogen or C₁₋₆-alkyl, and m is an integer from 0 to 4, W is a hydroxy protection group which can be hydrolytically cleaved under acidic conditions, and n is an integer from 1 to 3, with the proviso that n and m together are not greater than 5.

Particularly preferred, among other hydroxy protection groups, suitable hydroxy protection groups W is selected from
(a) -C(R⁵)(CH₂R⁶)-O-CH₂R⁷, wherein
   R⁵ is hydrogen or C₁₋₆-alkyl,
   R⁶ is selected from hydrogen, C₁₋₆-alkyl and C₃₋₆-cycloalkyl, and R⁷ is selected from hydrogen, C₁₋₆-alkyl, C₃₋₆-cycloalkyl and aryl, each alkyl, cycloalkyl or aryl of R⁵, R⁶ and R⁷ optionally and independently being substituted with one or more halogen atoms;
   or R⁶ and R⁷ together are -CH₂- or -(CH₂₎₂- and thus are part of an 5- or 6-membered heterocyclic ring, or
(b) -SiR⁸R⁹R¹⁰, wherein R⁸, R⁹ and R¹⁰ are independently selected from C₁₋₆-alkyl, C₃₋₆-cycloalkyl, aryl and aralkyl, or
   -(CₚH₂ₚ)-Z, wherein p is an integer from 1 to 6 and Z is H or -SR¹¹, wherein R¹¹ is selected from C₁₋₆-alkyl, C₃₋₆-cycloalkyl, aryl and aralkyl, or Z is -NR¹²R¹³, wherein R¹² and R¹³ are independently selected from C₁₋₆-alkyl, C₃₋₆-cycloalkyl, aryl and aralkyl, or R¹² and R¹³ together form a C₄₋₆-alkylene group.

In addition to the dicarbonyl compounds of formula II, also the oxime compounds of formula IV are new. Thus, claimed is a compound of formula 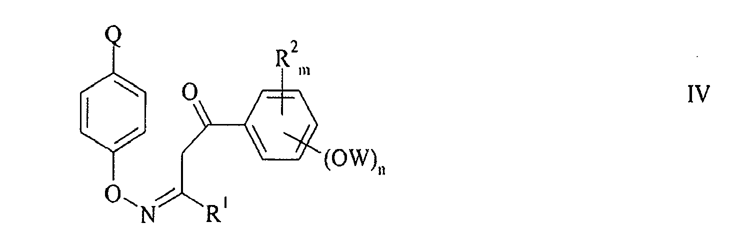 wherein R¹ is selected from C₁₋₆-alkyl, C₃₋₆-cycloalkyl and aralkyl,
R² at each occurrence independently is halogen or C₁₋₆-alkyl, and m is an integer from 0 to 4,
W is a hydroxy protection group which can be hydrolytically cleaved under acidic conditions, and n is an integer from 1 to 3, with the proviso that n and m together are not greater than 5.

More specifically claimed is a compound of formula IV,
wherein W at each occurrence is selected from
(a) -C(R⁵)(CH₂R⁶)-O-CH₂R⁷, wherein
   R⁵ is hydrogen or C₁₋₆-alkyl,
   R⁶-is selected from hydrogen, C₁₋₆-alkyl and C₃₋₆-cycloalkyl, and R⁷ is selected from hydrogen, C₁₋₆-alkyl, C₃₋₆-cycloalkyl and aryl, each alkyl, cycloalkyl or aryl of R⁵, R⁶ and R⁷ optionally and independently being substituted with one or more halogen atoms;
   or R⁶ and R⁷ together are -CH₂- or -(CH₂)₂- and thus are part of an 5- or 6-membered heterocyclic ring, or
(b) -SiR⁸R⁹R¹⁰, wherein R⁸, R⁹ and R¹⁰ are independently selected from C₁₋₆-alkyl,
   C₃₋₆-cycloalkyl, aryl and aralkyl, or
(c) -(CₚH₂ₚ)-Z, wherein p is an integer from 1 to 6 and Z is H or -SR¹¹, wherein R¹¹ is selected from C₁₋₆-alkyl, C₃₋₆-cycloalkyl, aryl and aralkyl, or Z is -NR¹²R¹³, wherein R¹² and R¹³ are independently selected from C₁₋₆-alkyl, C₃₋₆-cycloalkyl, aryl and aralkyl, or R¹² and R¹³ together form a C₄₋₆-alkylene group.

In a further preferred embodiment compounds of formula IV are selected from the group consisting of 1-(4-alkoxyphenyl)-3-(4-nitrophenyl-1-oxyimino)-heptane-1-one and 1-(4-hydroxyphenyl)-3-(4-nitrophenyl-1-oxyimino)-heptane-1-ones of formula 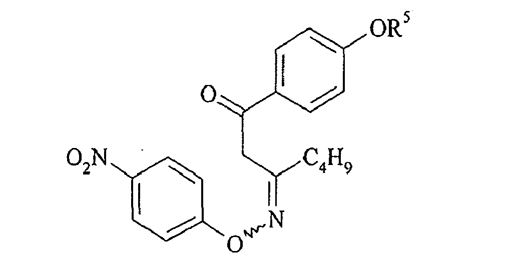 wherein Q is NO₂, R¹ is *n*-C₄H₉, Y is W is -(CₚH₂ₚ)-Z, wherein p is 1 to 6, Z is H, m is 0 and n is 1.

More preferably the compound of formula IV is 3-(4-nitrophenyl-1-oxyimino)-1-[4-[(3-*N*,*N*-di-propylamino)propyl-1-oxy]phenyl]-heptan-1-one 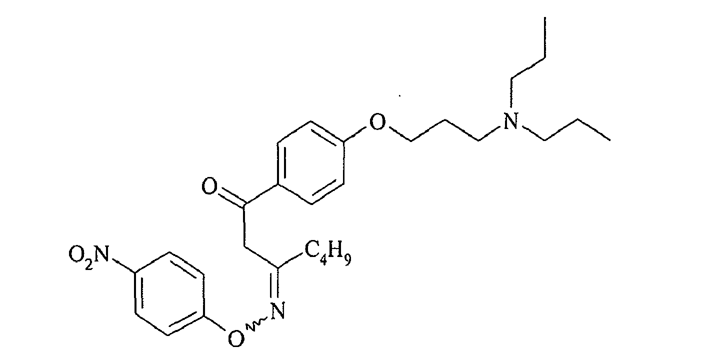 wherein Q = NO₂, R¹ = *n*-C₄H₉, Y = W = -(CₚH₂ₚ)-Z, wherein p = 3, Z = NR¹²R¹³, wherein R¹² = R¹³ = *n*-C₄H₉, m is 0 and n is 1.

### Examples

### Example 1: 1-[4-(1-Ethoxy-ethoxy)-phenyl]-ethanone (4-EEO-acetophenone, formula V: Y = W = -C(R⁵)(CH₂R⁶)-O-CH₂R⁷, R⁶ = R⁷ = H, R⁸ = CH₃, m = 0, n = 1)

4-Hydroxyacetophenone (4.1 g, 30 mmol) and ethyl vinyl ether (4.3 mL, 45 mmol) were dissolved in ethyl acetate (20 mL), which was acidified by addition of 0.5 mL of an ethyl acetate solution saturated with HCl gas, and then stirred for 2.5 h at room temperature. Sodium carbonate (1.0 g, 10 mmol) was added into the flask. After stirring for 20 min, the solution was filtered through a Celite^{®} pad. Triethylamine (0.5 mL, 3.5 mmol) was added to neutralize the filtrate, which was concentrated at 45 °C to give a light yellow oil (6.2 g, yield: 97%, HPLC purity at 254 nm: 91%).

### Example 2: 1-[4-(1-Ethoxy-ethoxy)-phenyl]-ethanone (Formula V: Y = W = -C(R⁵)(CH₂R⁶)-O-CH₂R⁷ with R⁵ = R⁶ = H, R⁷ = CH₃; m = 0, n = 1)

4-Hydroxyacetophenone (274 g, 2.01 mol) and THF (1.6 L) were introduced under nitrogen in a 6 L vessel and the mixture was cooled to 5 °C. During 6 h at 5 °C to this solution were added simultaneously a solution of ethyl vinyl ether (203.3 g, 2.817 mol) in THF (0.6 L) and a solution of methanesulfonic acid (1.2 g, 12.5 mmol) in THF (0.2 L). Then, the reaction mixture stirred within 3 h at 5 °C. The reaction was quenched by addition of triethylamine (2.53 g, 25 mmol). The resulting mixture was concentrated by vacuum distillation to remove THF and volatile matters, affording the product in quantitative yield as a light yellow oil (434.8 g).

### Example 3: 1-(4-Methoxy-phenyl)-heptane-1,3-dione (Formula II: R¹ = n-C₄H₉, Y = -(CₚH₂ₚ)-Z with p = 1, Z = H; m = 0, n = 1)

4-Methoxyacetophenone (3.0 g, 20 mmol) and methyl valerate (3.3 g, 28 mmol) were introduced into a 100 ml 3-necked flask under nitrogen. Then isobutyronitrile (31 g) and potassium *tert-*butylate (3.3 g, 28 mmol) were added. The suspension was heated to 87 °C, forming a slightly turbid solution. A first sample was taken for GC analysis after 30 min, showing nearly complete conversion. The reaction mixture was cooled to 10 °C, and the pH adjusted to 6.4 with 11.8% aqueous sulfuric acid. Water (20 mL) and ethyl acetate (20 mL) were added and the phases were separated. The yellow organic phase was washed with water (15 mL) and concentrated under reduced pressure (40-45 °C, to 11 mbar). 5 g of orange oil was obtained. HPLC purity : 86.5%

### Example 4: 1-(4-Methoxy-phenyl)-heptane-1,3-dione (Formula II: R¹ = n-C₄H₉, Y = -(CₚH₂ₚ)-Z with p = 1, Z = H; m = 0, n = 1)

1-[4-(1-Ethoxy-ethoxy)-phenyl]-ethanone (4-EEO-acetophenone, 400 g) and methyl valerate (346 g) were introduced in a 6 L glass reactor under nitrogen and cooled to 10 to 12 °C. Potassium *tert*-butylate (361.9 g) was added under stirring by portions within 1 h, keeping the inner temperature of the mixture below 15 °C. After complete addition the mixture was allowed to warm up slowly to 24 °C and kept stirring at that temperature for 4 h (overall 7 h). Toluene (1.838 L) and methanol (368 mL) were added, followed by conc. sulfuric acid (239 g) while keeping the temperature at about 24 °C. After stirring for further 30 min, water (2.638 L) was added, followed by 7% aq. NaOH (790.6 g) to neutralize the reaction mixture (pH 5.6). The layers were separated. The organic phase was washed with water (460 mL). From the organic phase the product was then extracted into the aqueous phase by adding 7% NaOH solution (2.0 kg, final pH = 12.9), and the organic phase was washed with water (460 mL). The combined aqueous layers were acidified with 50% sulfuric acid (362 g) to pH 5.6 and the product extracted into toluene (2000 mL). The toluene extract was washed with water (460 mL) and concentrated under reduced pressure (40 to 45 °C, 110 to 33 mbar) to afford the diketone as a reddish solution (Assay (NMR): 22.2%, Yield: 89.4% based on 4-hydroxyacetophenone).

### Example 5: 1-(4-Hydroxy-phenyl)-heptane-1,3-dione (Formula II: R¹ = n-C₄H₉, Y = H, m = 0, n = 1)

1-[4-(1-Ethoxy-ethoxy)-phenyl]-ethanone (4.92 g, 23.6 mmol) and ethyl valerate (4.22 mL, 28.4 mmol) were dissolved in 1,4-dioxane (40 mL). Then, 60% sodium hydride (1.42 g, 3.55 mmol) was added. The mixture was flushed with nitrogen gas. The resulting yellow suspension was stirred at 70 °C for 3 h. Then of HCl (IN, aq., 20 mL) was added. The bi-phase solution was stirred at 60 °C for 0.5 h. After cooling, ethyl acetate (20 mL) was added. After phase separation, the organic phase was further washed with water (20 mL), dried over Na₂SO₄, and then concentrated at 50 °C. The resulting oil was purified on silica gel column with ethyl acetate and petroleum ether (1:5, v:v) to give 2.7 g product (yield: 52%, purity: 69.6%).

### Example 6: 1-(4-hydroxy-phenyl)-heptane-1,3-dione (Formula II: Y = H, R¹ = n-C₄H₉, m = 0, n = 1)

4-Hydroxyacetophenone (27.2 g, 0.2 mol) was dissolved in THF (165 mL). Then, the mixture was cooled down to 0 °C. A H₂SO₄:THF mixture (1:9, w:w, 0.8 g) and ethyl vinyl ether (28.8 g, 0.4 mol) were added. The reaction mixture was stirred at 0 °C for 1 h. Et₃N (0.3 g) was added to adjust the mixture at about pH 8. Then, the mixture was allowed to warm to room temperature (RT). The solvent was removed under vacuum to get a light yellow oil (45.2 g) which was dissolved in isobutyronitril (IBN, 265 mL) before addition of methyl valerate (32.5 g, 0.28 mol). The reaction mixture was cooled down to 0 °C, and then potassium *tert*-butylate (38 g, 338 mmol) was added. The mixture was heated to 95 °C and kept at that temperature for 1 h. Then, the reaction mixture was cooled down to 0 °C. Water (50 mL) was added and the mixture was stirred for 15 min. The pH of the mixture was adjusted to about 7 with 10% H₂SO₄ (about 85 g). After stirring for 10 min the phases were allowed to separate. EtOH (10mL) was added to speed up the separation. The mixture was cooled down to 0 °C and HCl solution (2 mL, 37%) was added. After addition and stirring at 0 °C for 15 min, the pH was again adjusted to about 7 with saturated NaHCO₃ solution (39 g). Water (30 mL) was added and phases were allowed to separate. The organic phase was separated, washed with water and dried under vacuum to get a yellow oil (48 g). The residue was dissolved in CH₂Cl₂ (50 g) and then cooled down to 0 °C. *n*-Hexane (35 g) was added slowly and the mixture was stirred at 0 °C for 30 min and filtered. The filter cake was washed with a CH₂Cl₂:*n*-hexane mixture (1:2, v:v:, 75 mL) and dried under vacuum to get a yellow solid (33.5 g, 96.7% HPLC purity, yield: 72%). The mother liquid was dried under vacuum to get yellow oil (10 g).

### Example 7: Oxime Intermediate (Formula IV: R¹ = n-C₄H₉; Y = W = -C(R⁵)(CH₂R⁶)-O-CH₂R⁷ with R⁵ = R⁶ = H, R⁷ = CH₃; m = 0, Q = NO₂, n = 1)

To a mixture of toluene (1000 g) and 1-(4-hydroxy-phenyl)-heptane-1,3-dione (222 g), toluene (492 mL) and acetic acid (216 mL) were added. The solution was heated to 35 °C, and O-(4-nitrophenyl)-hydroxylamine (177.5 g) were added in 5 portions over 1 h. After stirring for 6 h at 35 °C, hexane (2 L) were added. The resulting suspension was cooled to 0 to 5 °C and filtered. The filter cake was first washed with a cold toluene/hexane mixture (2:1, v:v, 330 mL), then with hexane (120 mL). After drying the filter cake under vacuum at 40 °C the oxime product (366 g) was obtained as a beige solid with 92.9% purity, assay (NMR) 94.0% (Yield: 85.6% based on 4-hydroxyacetophenone).

### Example 8: 1-(4-Hydroxy-phenyl)-heptane-1,3-dione 3-[O-(4-nitro-phenyl)-oxime (Oxime intermediate, formula IV: R¹ = n-C₄H₉; Y = H, m = 0, Q = NO₂, n = 1)

1-(4-Hydroxy-phenyl)-heptane-1,3-dione (12.4 g, 0.05 mol) and O-(4-nitro phenyl) hydroxylamine (8.3 g, 0.05 mol) were stirred in CH₂Cl₂ (40 mL). Acetic acid (12 mL) was added and the colour of the clear solution turned to brown. The mixture was stirred at RT for 16 h. After addition of *n*-hexane (60 mL) into the flask, the mixture was cooled down to 0 °C and filtered. The filter cake was then washed with *n*-hexane:CH₂Cl₂ (1:1, v:v, 40 mL). Drying under vacuum afforded a white solid (17.1 g, 99%HPLC purity, yield: 85%).

### Example 9: 1-(4-Hydroxy-phenyl)-heptane-1,3-dione 3-[O-(4-nitro-phenyl)-oxime (Formula IV: R¹ = n-C₄H₉; Y = H, m = 0, Q = NO₂, n = 1)

4-Hydroxyacetophenone (27.2 g, 0.2 mol) was dissolved in THF (165 mL) and cooled down to 0 °C. A H₂SO₄:THF mixture (1:9, w:w, 0.8 g) was added. Ethyl vinyl ether (28.8g, 0.4 mol) was added in about 45 min and the mixture stirred for 1h. Et₃N (0.3 g) was added to adjust the pH at about 8. Then the reaction mixture was allowed to warm up to RT. After removing solvent and volatile matter under vacuum a light yellow oil was obtained (45.5 g). The oil was dissolved in IBN (230 mL) and then methyl valerate (32.5 g, 0.28 mol) was added. The mixture was cooled down to 0 °C and then potassium *tert*-butylate (38 g, 0.338 mol) was added. After complete addition the mixture was heated to 95 °C and stirred at that temperature for 2 h. The mixture was cooled down to 0 °C, and then water (50 mL) was added and the mixture again stirred for 15 min. The pH was adjusted to about 6 by addition of 10% H₂SO₄ (99 g) and the mixture stirred for 10 min. Then, the mixture was cooled down to 0 °C and 37% HCl solution (2 mL) was added. The mixture was stirred at 0 °C for 15 min, then the pH was adjusted to about 6 with 10% NaOH solution (5.5 g). Water (50 mL) was added and the phases separated. After drying the organic phase under vacuum a yellow solid (49 g) was obtained. The residue was dissolved in CH₂Cl₂ (120 mL), and then hydroxylamine (30.3g, 0.2 mol) was added. The mixtue was stirred at RT for 16 h. Heptane (150 mL) was added and then the mixture was cooled down to 0 °C and stirred at that temperature for 1 h. After filtration, the filter cake was washed with a CH₂Cl₂/*h*-heptane mixture (6:7, v:v, 130 mL), then dried under vacuum to obtain an off-white solid (62 g, 99.5% HPLC purity, 86%yield).

### Example 10: 1-(4-Hydroxy-phenyl)-heptane-1,3-dione 3-[O-(4-nitro-phenyl)-oxime (Formula IV: R¹ = n-C₄H₉; Y = H, m = 0, Q = NO₂, n = 1)

4-Hydroxyacetophenone (13.6 g, 0.1 mol) was dissolved in THF (82.5 mL), the solution was cooled at 0 °C and 10% sulfuric acid in THF (0.4 g) was added. Ethyl vinyl ether (14.4 g, 0.2 mol) was dosed within 50 min while keeping the inner temperature below 5 °C until an in-process control (IPC) showed complete conversion. The solution was neutralized with triethylamine (3.0 g), and the solvent and volatile matters were removed by vacuum distillation to obtain an oily residue (22.6 g). The residue was dissolved in isobutyronitrile (133 mL), and methyl valerate (16.3 g) was added. The mixture was cooled to 0 °C, then potassium *tert*-butylate (19 g) was added over 10 min. After complete addition, the reaction mixture was heated to 87 °C (bath temp. 95 °C) for 2 h until IPC showed virtually complete conversion. The mixture was cooled down to 0 °C, and water (50 mL) was added. After stirring for 10 min at that temperature, the mixture separated. The mixture was adjusted to pH 7 by adding 10% sulfuric acid (43 g), and the layers were separated. To the separated organic phase was added 37% hydrochloric acid (1 mL) and the solution was stirred for 25 min. After complete deprotection, the solution was adjusted to pH 5 to 6 by addition of a saturated NaHCO₃ solution (19 g), stirred for 5 min.. The organic phase was separated and washed with saturated brine (50 mL), and finally evaporated under reduced vacuum to obtain the diketone (26.8 g) as a yellow oil. The crude diketone was dissolved in CH₂Cl₂ (60 mL), followed by acetic acid (20 mL) and O-(4-nitro-phenyl)hydroxylamine (13.8 g). The mixture was stirred overnight at room temperature (27 °C). Then, an IPC showed complete conversion. *n*-Hexane (70 mL) was added to the mixture, and after stirring at 0 °C for 1 h, the precipitated product was filtered and washed with a CH₂Cl₂:hexane mixture (2:3, v:v, 50 mL). After drying under vacuum, the oxime was obtained as an off-white solid (27.2 g, yield: 76.3% based on 4-hydroxyacetophenone).

### Example 11: 1-(2-butyl-5-nitro-benzofuran-3-yl)-1-(4-hydroxy-phenyl)-methanone (Formula I: R¹ = n-C₄H₉, Y = -(CₚH₂ₚ)-Z with Z = H and p = 1; n = 1, m = 0, Q = NO₂)

1-(4-Hydroxy-phenyl)-heptane-1,3-dione (0.22 g, 1 mmol) and O-(4-nitro-phenyl)-hydroxylamine (0.15 g, 1 mmol) were dissolved in acetic acid (2 mL). Then 30% of HBr solution in acetic acid (2 mL) was added. After stirring at RT for 1 h, the reaction mixture was poured into ice-water (15 mL). Ethyl acetate (20 mL) was added. The resulting mixture was neutralized using sodium carbonate (about 4 g). After phase separation, the organic phase was further washed with water (20 mL), dried over Na₂SO₄, and finally concentrated to obtain an brown oil 0.41 g (purity: 71%, corrected yield: 86%).

### Example 12: 1-(2-butyl-5-nitro-benzofuran-3-yl)-1-(4-hydroxy-phenyl)-methanone (SI-004, formula (I) with R¹ = n-C₄H₉, Y = -(CₚH₂ₚ)-Z with Z = H and p = 1; n = 1, m = 0, Q = NO₂, oxime rearrangement in formic acid)

The oxime intermediate of example 7 (5.2 g, assay 97%) was suspended in formic acid (75 mL) under nitrogen, and the mixture was heated to 75 °C for 2.5 h (IPC). The dark solution was concentrated under reduced pressure to half of its volume, then cooled to 20 °C and the organic matter allowed to crystallizing. After crystallization, water (7.5 mL) was added dropwise, the suspension was gently cooled to 0 °C and stirred at that temperature for 30 min before filtration. The filter cake was washed with a water:formic acid mixture (1:1, v:v, 4 mL) and dried under vacuum at 40 °C. The product (SI-004, 3.73 g) was obtained as a white solid (purity : 99.9%, assay: 100%, yield: 75.5%).

### Example 13: SI-004 (oxime rearrangement promoted by trifluoroacetic acid)

The oxime intermediate of example 7 (5.07 g, assay 97%) was suspended in formic acid (73.2 g) under nitrogen, trifluoroacetic acid (7.2 g) was added, and the mixture was heated to 45 °C for 5 h (IPC). The dark solution was concentrated under reduced pressure to about 40 mL, then cooled to 20 °C for crystallization. After crystallization, water (12 mL) was added dropwise, the suspension was gently cooled to 0 °C and stirred at that temperature for 30 min before filtration. The filter cake was washed with a water:formic acid mixture (1:1, v:v, 4 mL) and dried under vacuum at 48 °C. SI-004 (4.17 g) was obtained as a white to beige solid (purity: 99.9%, assay 100%, yield: 87%).

### Example 14: SI-004

Example 13 was performed on 350 g scale. SI-004 (257.9 g) was obtained as a white powder with 99.3% purity (yield: 84.6%).

### Example 15: SI-004 (oxime rearrangement promoted by BF₃)

The oxime intermediate of example 7 (5.06 g, assay 97%) were suspended in formic acid (60 mL) under nitrogen, a solution of BF₃ etherate (2.0 g) in formic acid (15 mL) was added dropwise over I h at 20 °C. Then, the mixture was stirred for 8 h at 20 to 25 °C. BF₃ was quenched with triethylamine (1.5 g), and the mixture was concentrated under reduced pressure to 40 mL, and then cooled to 20 °C for crystallization. After crystallization, water (10 mL) was added dropwise, and then the mixture was slowly cooled to 0 °C and stirred at that temperature for 30 min before filtration. The filter cake first was washed with a water:forrmic acid mixture (1:1, v:v, 5 mL), then with water (5 mL), and finally dried under vacuum at 48 °C. SI-004 (4.08 g) was obtained as a white to beige solid (purity: 99.1 %, yield: 85%). According to GC this product contained only 0.7 area-% of regioisomer.

### Example 15: SI-004 (thermally promoted oxime rearrangement)

The oxime intermediate of example 7 (70 g, 197 mmol) and Celite^{®} (7.0 g) were stirred in xylene (350 mL), heated to 100 °C and reacted at that temperature for 11 h. Then the reaction mixture was filtered at 100 °C the filter cake washed with CH₂Cl₂. The filter cake was dried under vacuum to obtain a brown solid of crude SI-004 (68 g, purity:93% by HPLC, yield: 96%). The crude SI-004 (23 g) was completely dissolved in EtOH (100 mL) at 60 °C and then water (80 mL) was added.

The mixture was seeded with pure SI-004 solid (0.3 g) and cooled down to 30 °C in 45 min. A lot of solid appeared. The temperature was raised to 38 °C, the mixture stirred at that temperature for 30 min. Then, the mixture was cooled down to 20 °C in 20 min, followed by a stand-by of 60 min. The solid was filtered and the filter cake washed with EtOH:H₂O (1:1, v:v, 50 mL), and dried under vacuum to obtain a light beige solid (18.8 g, purity: 99.5% by HPLC yield: 86%).

## Claims

1. A process for the preparation of a compound of formula 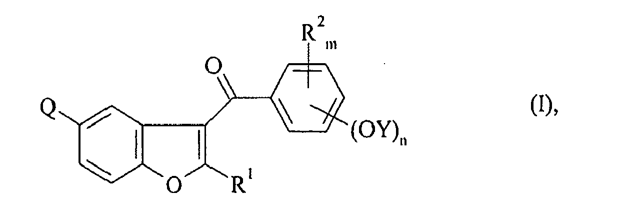 wherein R¹ is selected from C₁₋₆-alkyl, C₃₋₆-cycloalkyl and aralkyl,
R² at each occurrence independently is halogen or C₁₋₆-alkyl, and m is an integer from 0 to 4, Q is selected from halogen, -NO₂ and -NR³R⁴, wherein R³ and R⁴ are independently selected from hydrogen, C₁₋₆-alkyl, C₃₋₆-cycloalkyl, aryl, aralkyl, mesyl and tosyl, or wherein R³ and R⁴ together form a C₄₋₆-alkylene group,
Y at each occurrence is selected from hydrogen or a hydroxy protection group W that can be hydrolytically cleaved under acidic conditions, and n is an integer from 1 to 3, with the proviso that n and m together are not greater than 5,
comprising the steps of
(i) reacting a compound of formula 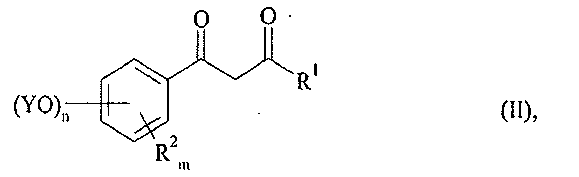 wherein R¹, R², Y, n and m are as defined above,
with a compound of formula 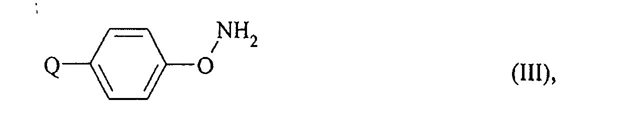 wherein Q is as defined above, or a salt thereof, optionally in the presence of an acid, to obtain a compound of formula 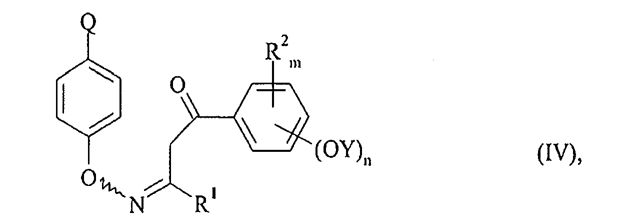 wherein R¹, R², Y, Q, n and in are as defined above, and
(ii) subjecting the compound of formula IV to an oxime rearrangement, optionally in the presence of an acid, to obtain the compound of formula I.

2. The process of claim 1, **characterized in that** W at each occurrence is
(a) -C(R⁵)(CH₂R⁶)-O-CH₂R⁷, wherein
R⁵ is hydrogen or C₁₋₆-alkyl,
R⁶ is selected from hydrogen, C₁₋₆-alkyl and C₃₋₆-cycloalkyl, and R⁷ is selected from hydrogen, C₁₋₆-alkyl, C₃₋₆-cycloalkyl and aryl, each alkyl, cycloalkyl or aryl of R⁵, R⁶ and
R⁷ optionally and independently being substituted with one or more halogen atoms;
or R⁶ and R⁷ together are -CH₂- or -(CH₂)₂- and thus are part of an 5- or 6-membered heterocyclic ring, or
(b) -SiR⁸R⁹R¹⁰, wherein R⁸, R⁹ and R¹⁰ are independently selected from C₁₋₆-alkyl, C₃₋₆-cycloalkyl, aryl and aralkyl, or
(c) -(CₚH₂ₚ)-Z, wherein p is an integer from 1 to 6 and Z is H or -SR¹¹, wherein R¹¹ is selected from C₁₋₆-alkyl, C₃₋₆-cycloalkyl, aryl and aralkyl, or Z is -NR¹²R¹³, wherein R¹² and R¹³ are independently selected from C₁₋₆-alkyl, C₃₋₆-cycloalkyl, aryl and aralkyl, or R¹² and R¹³ together form a C₄₋₆-alkylene group.

3. The process of claim 1 or 2, wherein the compound of formula IV is not isolated prior to step (ii).

4. The process of any of claims 1 to 3, wherein the oxime rearrangement of the compound of formula IV is carried out in the presence of an acid.

5. The use of a compound of formula IV as defined above in claims 1 to 5, wherein R¹, R² Q, n, and m are as defined in claim 1, for the preparation of a compound of formula I.

6. The use of a compound of formula I obtained by the process of any of claims 1 to 4, wherein R¹, R² Q, n, and m are as defined in claim 1, for the preparation of a medicament.

7. The use of claim 6, wherein the medicament is a medicament for therapeutic application in heart arrhythmia, angina pectoris and/or thrombosis.

8. A process for the preparation of a compound of formula 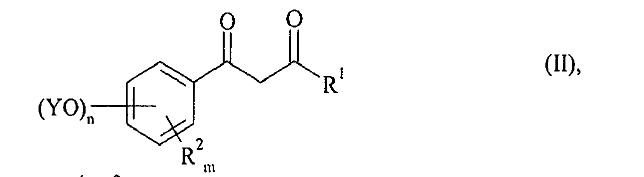 wherein R¹, R², Y, n and m are as defined in claim 1, comprising reacting a compound of formula 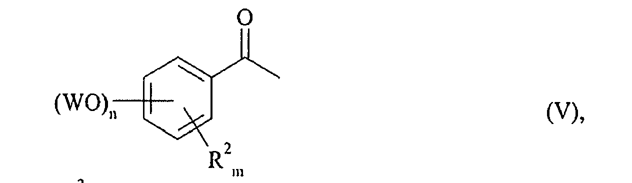 wherein R², W, n and m are as defined in claim 1, in the presence of a base, with a compound of formula 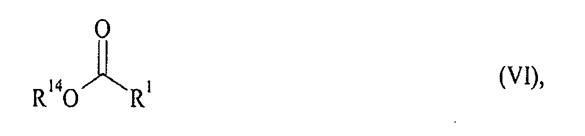 wherein R¹ is as defined in claim 1 and R¹⁴ is selected from the group consisting of C₁₋₆-alkyl, C₃₋₆-cycloalkyl and aralkyl, and
optionally hydrolytically cleaving W in the presence of an acid, to obtain a compound of formula II, wherein Y is hydrogen.

9. The process of claim 9, **characterized in that** W at each occurrence is
(a) -C(R⁵)(CH₂R⁶)-O-CH₂R⁷, wherein
R⁵ is hydrogen or C₁₋₆-alkyl,
R⁶ is selected from hydrogen, C₁₋₆-alkyl and C₃₋₆-cycloalkyl, and R⁷ is selected from hydrogen, C₁₋₆-alkyl, C₃₋₆-cycloalkyl and aryl, each alkyl, cycloalkyl or aryl of R⁵, R⁶ and
R⁷ optionally and independently being substituted with one or more halogen atoms;
or R⁶ and R⁷ together are -CH₂- or -(CH₂)₂- and thus are part of an 5- or 6-membered heterocyclic ring, or
(b) -SiR⁸R⁹R¹⁰, wherein R⁸, R⁹ and R¹⁰ are independently selected from C₁₋₆-alkyl, C₃₋₆-cycloalkyl, aryl and aralkyl, or
(c) -(CₚH₂ₚ)-Z, wherein p is an integer from 1 to 6 and Z is H or -SR¹¹, wherein R¹¹ is selected from C₁₋₆-alkyl, C₃₋₆-cycloalkyl, aryl and aralkyl, or Z is -NR¹²R¹³, wherein R¹² and R¹³ are independently selected from C₁₋₆-alkyl, C₃₋₆-cycloalkyl, aryl and aralkyl, or R¹² and R¹³ together form a C₄₋₆-alkylene group.

10. The process of claim 8 or 9. wherein the compound of formula 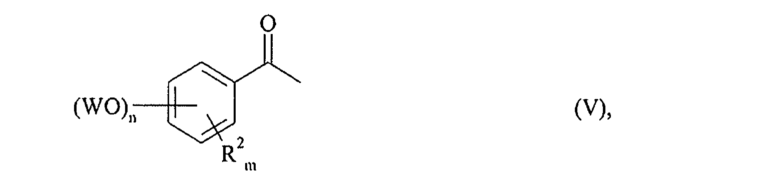 is prepared by reacting a compound of formula, 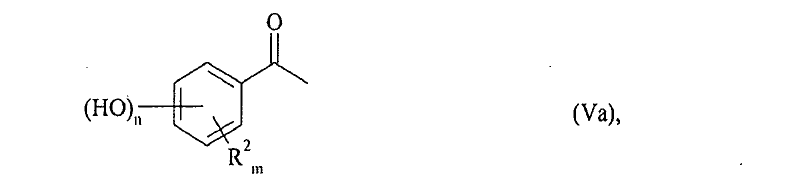 wherein R², n and m are as defined in claim 1, with at least n molar equivalents of
(a) a compound of formula 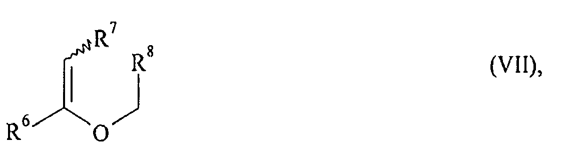 wherein R⁶, R⁷ and R⁸ are as defined in claim 2, to obtain a compound of formula 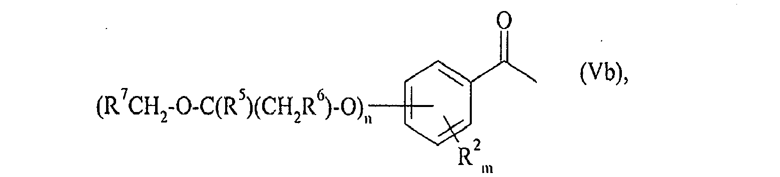 wherein R², R⁶, R⁷ and R⁸, m and n are as defined in claim 2, or
(b) a silylating agent comprising at least one group of the formula
R⁸R⁹R¹⁰Si- (VIII),
wherein R⁸, R⁹ and R¹⁰ are as defined in claim 2,
to obtain a compound of formula 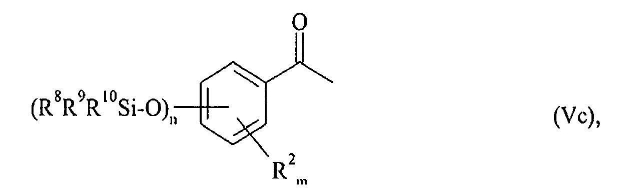 wherein R², R⁸, R⁹, R¹⁰, m and n are as defined in claim 2, or
(c) a compound of formula
T-(CₚH₂ₚ)-Z (IX),
wherein T is selected from mesyl, tosyl, chlorine, bromine and iodine, and p and Z are as defined in claim 2, to obtain a compound of formula 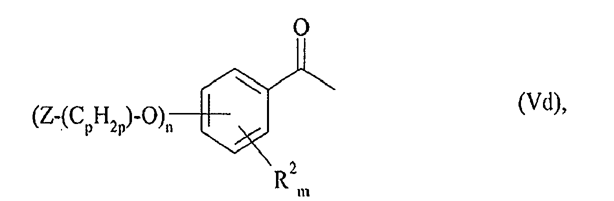
wherein R², Z, m, n and p are as defined in claim 2.

11. The process of claim 10, wherein the reaction is carried out at a temperature from 0 to 30 °C.

12. The process of claim 10 or 11, wherein the reaction is carried out in the presence of an acid catalyst.

13. The process of claim 8 or 9, wherein the hydrolytic cleavage of W is carried out in the presence of a diluted inorganic or organic proton acid, preferably selected from the group consisting of sulfuric acid, hydrochloric acid, formic acid and acetic acid to obtain the compound of formula II, wherein Y is hydrogen.

14. A compound of formula 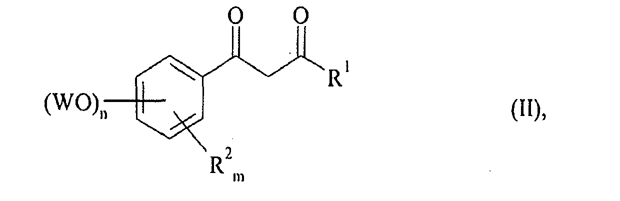 wherein R¹ is selected from C₁₋₆-alkyl, C₃₋₆-cycloalkyl and aralkyl,
R² at each occurrence independently is halogen or C₁₋₆-alkyl, and m is an integer from 0 to 4, W is a hydroxy protection group which can be hydrolytically cleaved under acidic conditions, and n is an integer from 1 to 3.

15. A compound of formula 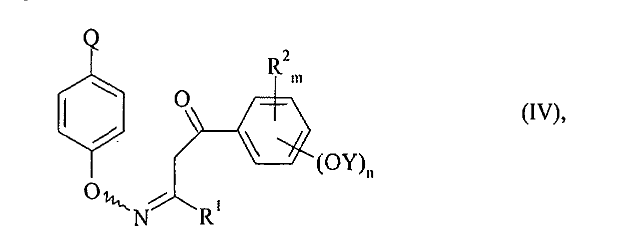 wherein R¹ is selected from C₁₋₆-alkyl, C₃₋₆-cycloalkyl and aralkyl,
R² at each occurrence independently is halogen or C₁₋₆-alkyl, and m is an integer from 0 to 4, Q is selected from halogen, -NO₂ and -NR³R⁴, wherein R³ and R⁴ are independently selected from hydrogen, C₁₋₆-alkyl, C₃₋₆-cycloalkyl, aryl, aralkyl, mesyl and tosyl, or wherein R³ and R⁴ together form a C₄₋₆-alkylene group,
Y at each occurrence is hydrogen or a hydroxy protection group W which can be hydrolytically cleaved under acidic conditions, and n is an integer from 1 to 3.
